# EUROPÄISCHE PATENTSCHRIFT

(11) **EP 3 439 538 B1**
(45) Veröffentlichungstag und Bekanntmachung des Hinweises auf die Patenterteilung: **29.07.2020**
(21) Anmeldenummer: 17712098.7
(22) Anmeldetag: 20.03.2017
(51) Int. Cl.: A61B 5/00, A61B 5/021, A61B 5/0285, A61B 5/0295, A61B 5/053

(54) **MESSVERFAHREN UND MESSVORRICHTUNG ZUR NICHT-INVASIVEN MESSUNG DER AORTALEN PULSWELLENGESCHWINDIGKEIT EINER MESSPERSON**
MEASUREMENT METHOD AND MEASURING DEVICE FOR NONINVASIVELY MEASURING THE AORTAL PULSE WAVE VELOCITY OF A MEASUREMENT SUBJECT
PROCÉDÉ DE MESURE ET DISPOSITIF DE MESURE POUR LA MESURE NON INVASIVE DE LA VITESSE D'ONDE PULSÉE DANS L'AORTE D'UN SUJET DE MESURE

(30) Priorität: 06.04.2016 DE 102016004462
(43) Veröffentlichungstag der Anmeldung: 13.02.2019
(73) Patentinhaber: Technische Hochschule Lübeck, 23562 Lübeck (DE)
(72) Erfinder: RYSCHKA, Martin, 23617 Stockelsdorf (DE); KUSCHE, Roman, 21075 Hamburg (DE)
(74) Vertreter: Wallinger Ricker Schlotter Tostmann
(86) Internationale Anmeldenummer: PCT/EP2017/056484
(87) Internationale Veröffentlichungsnummer: WO 2017/174334

(56) Entgegenhaltungen:
- EP-A2- 0 575 984
- EP-A2- 1 179 317
- US-A1- 2015 282 718
- DILPREET BUXI ET AL: "A survey on signals and systems in ambulatory blood pressure monitoring using pulse transit time", PHYSIOLOGICAL MEASUREMENT, INSTITUTE OF PHYSICS PUBLISHING, BRISTOL, GB, Bd. 36, Nr. 3, 19. Februar 2015 (2015-02-19), XP020280790, ISSN: 0967-3334, DOI: 10.1088/0967-3334/36/3/R1 [gefunden am 2015-02-19]
- IBRAHIM EL-SAYED H ET AL: "Measuring aortic pulse wave velocity using high-field cardiovascular magnetic resonance: comparison of techniques", JOURNAL OF CARDIOVASCULAR MAGNETIC RESONANCE, BIOMED CENTRAL LTD, LONDON UK, Bd. 12, Nr. 1, 11. Mai 2010 (2010-05-11), Seite 26, XP021079030, ISSN: 1532-429X, DOI: 10.1186/1532-429X-12-26

## Beschreibung

Die Erfindung betrifft ein Messverfahren und eine Messvorrichtung zur Messung der aortalen Pulswellengeschwindigkeit (pulse wave velocity, PWV) einer Messperson, d. h. der Ausbreitungsgeschwindigkeit von Druckwellen im Blut der Aorta, also der Hauptschlagader, der Messperson.

Ausgangspunkt für die Erfindung ist die hohe Zahl von Gefäßerkrankungen, für deren Diagnose und Prävention die Untersuchung der arteriellen Gefäßsteifigkeit mittels Pulswellenanalyse geeignet ist, da die Pulswellengeschwindigkeit ein deutlicher Indikator für die arterielle Gefäßsteifigkeit ist. Von besonders hohem medizinischem Interesse ist dabei die Pulswellengeschwindigkeit innerhalb der Aorta.

Unter anderem liefert diese Untersuchungsmethode eine prädiktive Größe für die Feststellung und Beurteilung kardiovaskulärer Ereignisse. Weiter liefert sie wesentliche Ergebnisse zur Bestimmung einer Therapie oder Therapieoptimierung.

Ein weiterer wesentlicher Vorteil diese Untersuchungsmethode gegenüber Methoden mit Eingriffen in den Körper ist die nicht-invasive Bestimmung des Aortendruckverlaufs und die Ableitung prädiktiver Parameter wie etwa, neben der Pulswellengeschwindigkeit, des Augmentationsindex.

Grundsätzlich kann die aortale Pulswellengeschwindigkeit aus der Zeitdifferenz des Auftretens der Pulswelle in der Aorta an zwei verschiedenen Stellen mit einem bekannten Abstand voneinander bestimmt werden, wobei der Anstoß der Pulswelle an die Arterienwand am Messort durch eine Kontraktion des Herzmuskels ausgelöst wird.

Herkömmliche Messmethoden zur Bestimmung der aortalen Pulswellengeschwindigkeit basieren u. a. auf einer Auswertung der Morphologie der detektierten Pulswelle oder auf einer Laufzeitabschätzung der Pulswelle durch Detektion der Pulswelle am Hals (Arteria carotis) und am Oberschenkel (Arteria femoralis).

Dilpreet Buxi et al., "A survey on signals and systems in ambulatory blood pressure monitoring using pulse transit time", Physiological Measurement, Institute Of Physics Publishing, Bristol, Vol. 36, No. 3 (2015) geben einen Überblick über verschiedene Verfahren zur Blutdrucküberwachung durch nicht-invasive Messung der aortalen Pulswellengeschwindigkeit einer Messperson und klassifizieren diese Verfahren hinsichtlich der Aspekte Kalibration der Pulswellenlauf- und -ankunftszeiten zum Blutdruck, Erhebung und Verarbeitung von physiologischen Signalen und Entwurf von voll integrierten Blutdruckmesssystemen.

Die US 2015/0282718 A1 verwendet einen Ballistokardiogramm-(BCG)-Sensor, um Herz- und Gefäßcharakteristiken eines Benutzers zu detektieren und eine BCG-Ausgabe bereitzustellen, welche für die detektierten kardiovaskulären Charakteristiken kennzeichnend ist.

In dem Verfahren gemäß der EP 1 179 317 A2 wird mit Hilfe eines Mikrofons zur Detektierung der Herztöne und einer Vorrichtung zur Detektion, ob die Person gerade ein- oder ausatmet, aus den Herztönen in den Zeitintervallen, in denen die Person einatmet, die Schließzeit der Aortenklappe ermittelt. Daraus und aus der Detektion einer Pulswelle in einer Arterie der Person wird dann die Pulswellengeschwindigkeit ermittelt.

El-Sayed H. Ibrahim et al., "Measuring aortic pulse wave velocity using high-field cardiovascular magnetic resonance: comparison of techniques", Journal Of Cardiovascular Magnetic Resonance, Biomed Central Ltd, London, Vol. 12, No. 1 (2010) vergleichen drei Verfahren zur Messung der Pulswellengeschwindigkeit durch geschwindigkeitskodierte kardiovaskuläre Magnetresonanz bei hoher Feldstärke.

Aus der EP 0 575 984 A2 ist ein nicht-invasives System zur Bestimmung der wichtigsten kardiorespiratorischen Parameter des menschlichen Körpers aus der aktiven Komponente einer integralen Bioimpedanzmessung des gesamten Körpers bekannt.

Der vorliegenden Erfindung liegt die Aufgabe zu Grunde, ein verbessertes Messverfahren und eine verbesserte Messvorrichtung zur Bestimmung der aortalen Pulswellengeschwindigkeit zu schaffen.

Diese Aufgabe wird durch das Messverfahren gemäß Anspruch 1 und durch die Messvorrichtung gemäß Anspruch 8 gelöst. Vorteilhafte Weiterbildungen der Erfindung sind in den Unteransprüchen enthalten.

Das erfindungsgemäße Messverfahren zur nicht-invasiven Messung der aortalen Pulswellengeschwindigkeit weist die folgenden Schritte auf:
- Anbringen wenigstens eines ersten Pulswellensensors an jeweils einer ersten Stelle auf der Hautoberfläche der Messperson im Bereich der Aorta,
- Anbringen wenigstens eines zweiten Pulswellensensors an jeweils einer zweiten Stelle, welche von jeder der ersten Stellen verschieden ist, auf der Hautoberfläche der Messperson im Bereich der Aorta,
- Anbringen wenigstens eines akustischen Aktors zur Erzeugung akustischer Signale an jeweils einer dritten Stelle, welche an oder im Bereich wenigstens einer der ersten Stellen liegt,
- Anbringen wenigstens eines akustischen Sensors zur Detektion akustischer Signale an jeweils einer vierten Stelle, welche an oder im Bereich wenigstens einer der zweiten Stellen liegt,
- Detektieren wenigstens einer Pulswelle in der Aorta der Messperson durch wenigstens einen der ersten Pulswellensensoren an der jeweiligen ersten Stelle und Bestimmen des Zeitpunkts der Detektion,
- Detektieren derselben Pulswelle durch wenigstens einen der zweiten Pulswellensensoren an der jeweiligen zweiten Stelle und Bestimmen des Zeitpunkts der Detektion,
- Erzeugen wenigstens eines akustischen Signals durch wenigstens einen der akustischen Aktoren an der jeweiligen dritten Stelle und Bestimmen des Zeitpunkts der Erzeugung,
- Detektieren desselben akustischen Signals durch wenigstens einen der akustischen Sensoren an der jeweiligen vierten Stelle und Bestimmen des Zeitpunkts der Detektion,
- Bestimmen einer Laufzeit wenigstens einer der Pulswellen zwischen wenigstens einer der ersten und wenigstens einer der zweiten Stellen,
- Bestimmen einer Laufzeit wenigstens eines der akustischen Signale zwischen wenigstens einer der dritten und wenigstens einer der vierten Stellen,
- Bestimmen eines Signallaufwegs des wenigstens einen akustischen Signals zwischen der wenigstens einen dritten Stelle und der wenigstens einen vierten Stelle aus der Laufzeit des wenigstens einen akustischen Signals,
- Bestimmen einer Pulswellengeschwindigkeit aus dem Signallaufweg des wenigstens einen akustischen Signals und der Laufzeit der wenigstens einen Pulswelle.

Der Begriff "Pulswellensensor" ist hierbei nicht notwendigerweise als ein einzelner punkt- oder flächenförmiger Sensor zu verstehen, sondern kann insbesondere auch mehrere einzelne Sensoren, insbesondere mehrere Einzelelektroden, aufweisen.

Der Begriff "Stelle auf der Hautoberfläche im Bereich der Aorta" bezeichnet im vorliegenden Kontext eine Stelle auf der Hautoberfläche, welche von der senkrechten Projektion der Aorta auf die Hautoberfläche bevorzugt höchstens 5 cm, weiter bevorzugt höchstens 3 cm und noch weiter bevorzugt höchstens 1 cm entfernt ist und besonders bevorzugt auf dieser Projektion liegt.

Entsprechend bezeichnet der Begriff "Stelle im Bereich wenigstens einer der ersten/zweiten Stellen" eine Stelle, welche von der betreffenden ersten bzw. zweiten Stelle bevorzugt höchstens 5 cm, weiter bevorzugt höchstens 3 cm und noch weiter bevorzugt höchstens 1 cm entfernt ist und besonders bevorzugt mit dieser ersten bzw. zweiten Stelle zusammenfällt.

Der Erfindung liegt der Gedanke zu Grunde, den Laufweg der Pulswelle indirekt über den Laufweg eines akustischen Signals zu bestimmen, indem die Pulswellensensoren und die akustischen Aktoren bzw. Sensoren jeweils an benachbarten oder an den gleichen Stellen angebracht werden. In diesem Fall kann der Laufweg der Pulswelle sehr gut durch den Laufweg des akustischen Signals approximiert werden.

Vorzugsweise erfolgt die Bestimmung der Pulswellengeschwindigkeit dann aus einer Division des Signallaufweges des akustischen Signals durch die Laufzeit der Pulswelle. Weiter vorzugsweise werden mehrere Einzelwerte für die Pulswellengeschwindigkeit, insbesondere aufgrund einer Erzeugung verschiedener Signale durch verschiedene Aktoren und/oder von Messungen durch verschiedene Sensoren, berechnet und der endgültige Wert der Pulswellengeschwindigkeit insbesondere als Mittelwert dieser Einzelwerte bestimmt.

Das akustische Signal breiten sich im Körper der Messperson als Körperschall mit Schallgeschwindigkeit aus, welche im menschlichen Körper ca. 1540 m/s beträgt. Vorzugsweise erfolgt daher die Bestimmung des Signallaufweges des akustischen Signals durch eine Multiplikation dieser Schallgeschwindigkeit mit der Signallaufzeit des akustischen Signals.

Das erfindungsgemäße Messverfahren hat mehrere Vorteile:
- Es lässt sich ein kostengünstiges Messsystem mit hoher Messgenauigkeit realisieren.
- Die von der Pulswelle innerhalb der gemessenen Differenzzeit zurückgelegte Stecke braucht nicht grob abgeschätzt oder manuell gemessen zu werden, sondern wird über die Laufzeit der akustischen Signale mit hoher Genauigkeit gemessen.
- Es sind eine zeitkontinuierliche Messung der Pulswellengeschwindigkeit sowie Langzeitmessungen möglich.

Das erfindungsgemäße Messverfahren sowie die erfindungsgemäße Messvorrichtung können unter anderem im Operationssaal, für die ambulante Pulswellengeschwindigkeitsmessung, für Langzeitmessungen sowie im Fitnessbereich eingesetzt werden.

Zusätzlich können mit dem erfindungsgemäßen Messverfahren auch noch weitere Vitalparameter gemessen werden.

In einer bevorzugten Ausführung der Erfindung sind wenigstens zwei erste Pulswellensensoren und/oder wenigstens zwei zweite Pulswellensensoren vorgesehen. Ebenso können vorzugsweise wenigstens zwei akustische Aktoren und/oder wenigstens zwei akustische Sensoren vorgesehen sein.

Durch die mehrfache Auslegung der Sensoren bzw. Aktoren lassen sich Messungen an unterschiedlichen, vorzugsweise nahe beieinander gelegenen Stellen vornehmen und die daraus gewonnenen Messwerte vorzugsweise mitteln, um genauere Werte für die Pulswellengeschwindigkeit zu erhalten.

In einer weiteren bevorzugten Ausführung der Erfindung sind wenigstens eine der ersten und wenigstens eine der dritten Stellen benachbart oder fallen zusammen, und/oder es sind wenigstens eine der zweiten und wenigstens eine der vierten Stellen benachbart oder fallen zusammen.

Dies bedeutet, dass zumindest einzelne Pulswellensensoren nahe bei oder gemeinsam mit akustischen Aktoren oder Sensoren auf der Hautoberfläche der Messperson angebracht werden. Dadurch ist zum einen eine Messung der Pulswellen und der akustischen Signale zwischen benachbarten oder den gleichen Stellen und damit eine besonders genaue Messung der Pulswellengeschwindigkeit gewährleistet. Zum anderen vereinfacht dies die Ausführung des Messverfahrens, da die Pulswellensensoren und die akustischen Aktoren bzw. Sensoren ggf. gemeinsam auf einem Trägermaterial befestigt und auf der Hautoberfläche der Messperson angebracht, insbesondere aufgeklebt, werden können.

In einer weiteren bevorzugten Ausführung der Erfindung erfolgt die Detektion der Pulswelle durch wenigstens einen der ersten und/oder durch wenigstens einen der zweiten Pulswellensensoren durch eine Bioimpedanzmessung.

Bei einer Bioimpedanzmessung wird die Impedanz, d. h. der Wechselstromwiderstand, bestimmter Körperbestandteile, im vorliegenden Fall von Blutgefäßen und insbesondere der Aorta, gemessen. Aus dem zeitlichen Verlauf der Impedanz kann dabei auch auf das Auftreten einer Pulswelle in der Aorta geschlossen werden.

Bei der Bioimpedanzmessung werden typischerweise vier Elektroden auf der Hautoberfläche der Messperson angebracht. Dabei wird über die zwei äußeren (distalen) Elektroden ein elektromagnetisches Feld im Körper der Messperson aufgebaut und über zwei weitere, innere (proximale) Elektroden der Spannungsabfall und die Phasenverschiebung der Signalspannung gemessen.

Das distale und das proximale Elektrodenpaar werden vorzugsweise derart auf der Hautoberfläche der Messperson angebracht, dass die Verbindungsstrecke zwischen den distalen und/oder den proximalen Elektroden die Projektion der Aorta auf die Hautoberfläche schneidet. Die erste bzw. die zweite Stelle, an der der jeweilige Pulswellensensor auf der Hautoberfläche der Messperson angebracht ist, entspricht dann vorzugsweise diesem Schnittpunkt oder einem dieser Schnittpunkte.

Mit den bei der Bioimpedanzmessung genutzten Elektroden lässt sich insbesondere zusätzlich auch ein Elektrokardiogramm aufzeichnen.

Die Elektroden können auch mit weiteren Sensoren, insbesondere mit thermischen und/oder optischen Sensoren, ausgestattet werden.

Weiterhin müssen bei einer Bioimpedanzmessung keine Druckmanschetten verwendet werden, welche zu einer Manipulation des Arteriensystems während des Messvorgangs führen können. Die Elektroden für die Bioimpedanzmessung beeinflussen dagegen das Arteriensystem mechanisch nicht und beeinträchtigen außerdem den Patienten weniger. Weiterhin erlauben sie eine Messung auch ohne speziell geschultes Personal.

Die Elektroden bei der Bioimpedanzmessung können in einfacher Weise als Klebeelektroden ausgebildet sein.

In einer weiteren bevorzugten Ausführung der Erfindung liegt wenigstens eine der ersten Stellen in der Herzregion, insbesondere im Bereich des Aortenbogens, der Messperson.

In einer weiteren bevorzugten Ausführung der Erfindung liegt wenigstens eine der zweiten Stellen im Bereich eines Oberschenkels der Messperson, insbesondere im oberen Bereich eines Oberschenkels.

Auf diese Weise sind ein genügend langer Signallaufweg des akustischen Signals und ein genügend langer Laufweg der Pulswelle gegeben, um genaue Messergebnisse für die Pulswellengeschwindigkeit zu erzielen.

Sofern mit dem erfindungsgemäßen Verfahren die Pulswelle zu Beginn der Aorta und nach Austritt aus der Aorta detektiert wird, lässt sich aus der Morphologie der Pulswelle auch die Systemfunktion der Aorta ermitteln.

Eine erfindungsgemäße Messvorrichtung weist die folgenden Komponenten auf, wobei sich durch Merkmale, die dem erfindungsgemäßen Verfahren entsprechen, auch entsprechende Wirkungen und Vorteile ergeben:
- wenigstens ein erster Pulswellensensor zur Anbringung an jeweils einer ersten Stelle auf der Hautoberfläche der Messperson im Bereich der Aorta,
- wenigstens ein zweiter Pulswellensensor zur Anbringung an jeweils einer zweiten Stelle, welche von jeder der ersten Stellen verschieden ist, auf der Hautoberfläche der Messperson im Bereich der Aorta,
- wenigstens ein akustischer Aktor zur Erzeugung akustischer Signale zur Anbringung an jeweils einer dritten Stelle, welche an oder im Bereich wenigstens einer der ersten Stellen liegt,
- wenigstens ein akustischer Sensor zur Detektion akustischer Signale zur Anbringung an jeweils einer vierten Stelle, welche an oder im Bereich wenigstens einer der zweiten Stellen liegt,
- eine erste Auswerteeinrichtung zur Bestimmung der Zeitpunkts einer Detektion wenigstens einer Pulswelle durch wenigstens einen der ersten Pulswellensensoren an der jeweiligen ersten Stelle,
- eine zweite Auswerteeinrichtung zur Bestimmung der Zeitpunkts einer Detektion derselben Pulswelle durch wenigstens einen der zweiten Pulswellensensoren an der jeweiligen zweiten Stelle,
- eine dritte Auswerteeinrichtung zur Bestimmung des Zeitpunkts einer Erzeugung wenigstens eines akustischen Signals durch wenigstens einen der akustischen Aktoren an der jeweiligen dritten Stelle,
- eine vierte Auswerteeinrichtung zur Bestimmung des Zeitpunkts einer Detektion desselben akustischen Signals durch wenigstens einen der akustischen Sensoren an der jeweiligen vierten Stelle,
- eine erste Laufzeitbestimmungseinrichtung zur Bestimmung einer Laufzeit wenigstens einer der Pulswellen zwischen wenigstens einer der ersten und wenigstens einer der zweiten Stellen,
- eine zweite Laufzeitbestimmungseinrichtung zur Bestimmung einer Laufzeit wenigstens eines der akustischen Signale zwischen wenigstens einer der dritten und wenigstens einer der vierten Stellen,
- eine Signallaufwegsbestimmungseinrichtung zur Bestimmung eines Signallaufwegs des wenigstens einen akustischen Signals zwischen der wenigstens einen dritten und der wenigstens einen vierten Stelle aus der Laufzeit des wenigstens einen akustischen Signals,
- eine Pulswellengeschwindigkeitsbestimmungseinrichtung zur Bestimmung einer Pulswellengeschwindigkeit aus dem Signallaufweg des wenigstens einen akustischen Signals und der Laufzeit der wenigstens einen Pulswelle.

In einer bevorzugten Ausführung der Erfindung sind wenigstens zwei erste Pulswellensensoren und/oder wenigstens zwei zweite Pulswellensensoren vorgesehen.

In einer weiteren bevorzugten Ausführung der Erfindung sind wenigstens zwei akustische Aktoren und/oder wenigstens zwei akustische Sensoren vorgesehen.

In einer weiteren bevorzugten Ausführung der Erfindung ist wenigstens ein erster Pulswellensensor und/oder wenigstens ein zweiter Pulswellensensor als Elektrode, insbesondere als Textil- oder Kunststoffelektrode, ausgebildet.

Dies erlaubt eine besonders einfache und bequeme Anbringung auf der Hautoberfläche der Messperson.

In einer weiteren bevorzugten Ausführung der Erfindung sind wenigstens ein erster Pulswellensensor und wenigstens ein akustischer Aktor und/oder wenigstens ein zweiter Pulswellensensor und wenigstens ein akustischer Sensor jeweils zur gemeinsamen Anbringung auf der Hautoberfläche der Messperson ausgebildet.

Dies bewirkt, dass wenigstens eine der ersten und wenigstens eine der dritten Stellen benachbart sind oder zusammenfallen und/oder dass wenigstens eine der zweiten und wenigstens eine der vierten Stellen benachbart sind oder zusammenfallen.

In einer weiteren bevorzugten Ausführung der Erfindung ist wenigstens einer der ersten und/oder wenigstens einer der zweiten Pulswellensensoren ein Bioimpedanzsensor.

Es sei angemerkt, dass die Erfindung auch für Messungen an Tieren Verwendung finden kann, der Begriff "Messperson" umfaßt somit auch Tiere.

Weitere Vorteile, Merkmale und Anwendungsmöglichkeiten der vorliegenden Erfindung ergeben sich aus der nachfolgenden Beschreibung im Zusammenhang mit den Figuren. Es zeigen:
- Fig. 1: ein Prinzipschaubild einer erfindungsgemäßen Messvorrichtung und
- Fig. 2: eine vereinfachte Darstellung des menschlichen Arteriensystems mit einer Kennzeichnung der für das erfindungsgemäße Messverfahren angebrachten Elektroden und der für das Verfahren maßgeblichen Strecken.

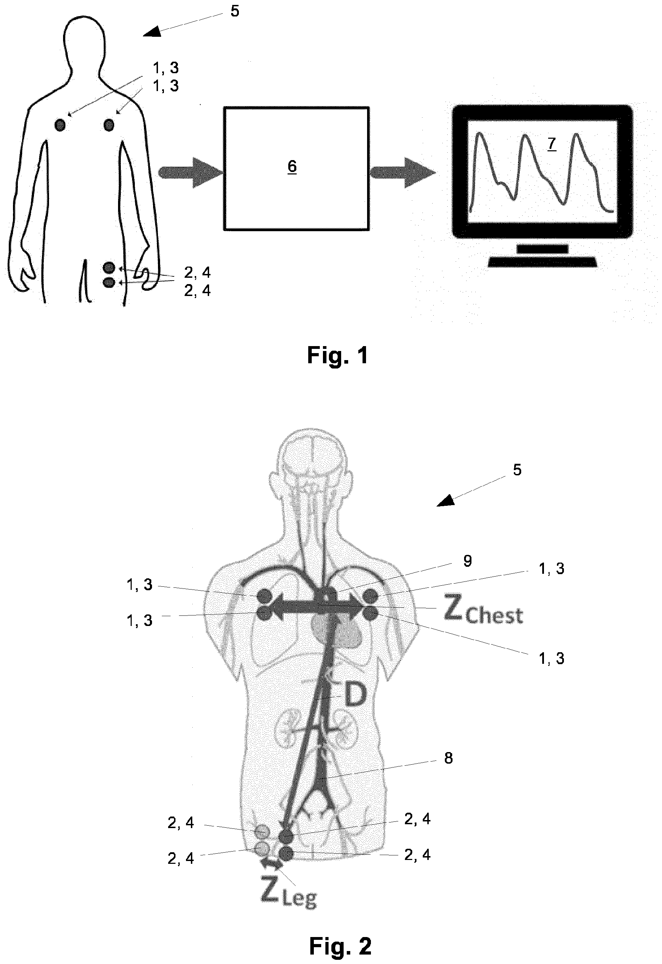

- Fig. 1: zeigt schematisch ein Prinzipschaubild einer erfindungsgemäßen Mess-

vorrichtung, wobei links eine Messperson 5 mit auf dessen Hautoberfläche befestigten Sensoren und Aktoren, in der Mitte eine Mess- und Auswerteeinrichtung 6 und rechts eine Anzeigeeinrichtung 7 abgebildet sind. Die Fluss der Signale und Informationen in dem Messverfahren ist durch die beiden dicken Pfeile angedeutet.

An der Messperson sind im linken sowie im rechten Bereich der Brust Elektroden für eine Bioimpedanzmessung angebracht, vorzugsweise aufgeklebt, welche gemeinsam einen ersten Pulswellensensor 1 bilden. Wenigstens eine der Elektroden ist mit einem akustischen Aktor, insbesondere einem Vibrationsgeber oder Lautsprecher, kombiniert. Entsprechende Elektroden, welche gemeinsam einen zweiten Pulswellensensor 2 bilden und von denen wenigstens eine mit einem akustischen Sensor, insbesondere einem Mikrofon, kombiniert ist, sind im oberen Bereich des linken Oberschenkels der Messperson angebracht.

Wie in Fig. 2 zu erkennen ist, misst bei der Ausführung des erfindungsgemäßen Messverfahrens der erste Pulswellensensor 1 die Bioimpedanz Z_{Chest} an einer ersten Stelle an der Aorta 8 im Herzbereich, insbesondere am Aortenbogen 9, der Messperson 5. Der zweite Pulswellensensor 2 misst gleichzeitig die Bioimpedanz Z_{Leg} an einer zweiten Stelle an der Aorta 8 im Oberschenkel der Messperson 5.

Die Messwerte werden an die Mess- und Auswerteeinrichtung 6 weitergegeben. Diese bestimmt aus den Messwerten den zeitlichen Verlauf einer Pulswelle an der ersten sowie an der zweiten Stelle, wie er beispielhaft in Fig. 1 auf dem Bildschirm der Anzeigevorrichtung 7 für eine der beiden Stellen dargestellt ist. Daraus bestimmt die Mess- und Auswerteeinrichtung 6 die beiden Zeitpunkte, an denen die Pulswelle an der ersten bzw. an der zweiten Stelle den größten Druck auf die Aorta 8 ausübt, d. h. die Peaks der Pulswelle, sowie die Zeitdifferenz zwischen diesen beiden Zeitpunkten und damit die Laufzeit der Pulswelle zwischen der ersten und der zweiten Stelle.

Weiterhin gibt der akustische Aktor 3 ein akustisches Signal einer bestimmten Frequenz ab, welches sich im Körper der Messperson mit einer Schallgeschwindigkeit von ca. 1540 m/s ausbreitet und von dem akustischen Sensor 4 detektiert wird. Auch das erzeugte und das detektierte akustische Signal werden von dem akustischen Aktor 3 bzw. von dem akustischen Sensor 4 an die Mess- und Auswerteeinrichtung 6 weitergegeben bzw. im Falle der Erzeugung des akustischen Signals von einer (nicht dargestellten) Steuereinheit für die Signalerzeugung der Mess- und Auswerteeinrichtung 6 mitgeteilt.

Die Mess- und Auswerteeinrichtung 6 bestimmt den Zeitpunkt der Erzeugung und den Zeitpunkt der Detektion des akustischen Signals und berechnet daraus die Zeitdifferenz zwischen diesen beiden Zeitpunkten sowie durch Multiplikation dieser Zeitdifferenz mit der Schallgeschwindigkeit den Signallaufweg des akustischen Signals und damit den Abstand D zwischen dem akustischen Aktor 3 und dem akustischen Sensor 4.

Da der akustische Aktor 3 gemeinsam mit einer Elektrode des ersten Pulswellensensors 1 und der akustische Sensor 4 gemeinsam mit einer Elektrode des zweiten Pulswellensensors 2 angebracht ist, entspricht der Abstand D näherungsweise dem Laufweg der Pulswelle zwischen der ersten und der zweiten Stelle.

Durch Division des Abstands D durch die Laufzeit der Pulswelle berechnet die Mess- und Auswerteeinrichtung 6 schließlich die Pulswellengeschwindigkeit. Diese wird vorzugsweise gespeichert und/oder auf der Anzeigeeinrichtung 7, bei welcher es sich vorzugsweise um einen Bildschirm oder Drucker handelt, ausgegeben.

### Bezugszeichenliste

- 1: Erster Pulswellensensor
- 2: Zweiter Pulswellensensor
- 3: Akustischer Aktor
- 4: Akustischer Sensor
- 5: Messperson
- 6: Mess- und Auswerteeinrichtung
- 7: Anzeigeeinrichtung
- 8: Aorta
- 9: Aortenbogen

- Z_{Chest}: Impedanz im Brustbereich
- Z_{Leg}: Impedanz im Oberschenkelbereich
- D: Länge des Signallaufwegs

## Patentansprüche

1. Messverfahren zur nicht-invasiven Messung der aortalen Pulswellengeschwindigkeit einer Messperson (5), d. h. der Ausbreitungsgeschwindigkeit von Druckwellen im Blut der Aorta (8) der Messperson (5), mit den Schritten:
- Anbringen wenigstens eines ersten Pulswellensensors (1) an jeweils einer ersten Stelle auf der Hautoberfläche der Messperson (5) im Bereich der Aorta (8), wobei die erste Stelle von der senkrechten Projektion der Aorta (8) auf die Hautoberfläche höchstens 5 cm, bevorzugt höchstens 3 cm und weiter bevorzugt höchstens 1 cm entfernt ist und besonders bevorzugt auf dieser Projektion liegt,
- Anbringen wenigstens eines zweiten Pulswellensensors (2) an jeweils einer zweiten Stelle, welche von jeder der ersten Stellen verschieden ist, auf der Hautoberfläche der Messperson (5) im Bereich der Aorta (8), wobei die zweite Stelle von der senkrechten Projektion der Aorta (8) auf die Hautoberfläche höchstens 5 cm, bevorzugt höchstens 3 cm und weiter bevorzugt höchstens 1 cm entfernt ist und besonders bevorzugt auf dieser Projektion liegt,
- Anbringen wenigstens eines akustischen Aktors (3) zur Erzeugung akustischer Signale an jeweils einer dritten Stelle, welche an oder im Bereich wenigstens einer der ersten Stellen liegt, wobei die dritte Stelle von der betreffenden ersten Stelle höchstens 5 cm, bevorzugt höchstens 3 cm und weiter bevorzugt höchstens 1 cm entfernt ist und besonders bevorzugt mit dieser ersten Stelle zusammenfällt,
- Anbringen wenigstens eines akustischen Sensors (4) zur Detektion akustischer Signale an jeweils einer vierten Stelle, welche an oder im Bereich wenigstens einer der zweiten Stellen liegt, wobei die vierte Stelle von der betreffenden zweiten Stelle höchstens 5 cm, bevorzugt höchstens 3 cm und weiter bevorzugt höchstens 1 cm entfernt ist und besonders bevorzugt mit dieser zweiten Stelle zusammenfällt,
- Detektieren wenigstens einer Pulswelle in der Aorta (8) der Messperson (5) durch wenigstens einen der ersten Pulswellensensoren (1) an der jeweiligen ersten Stelle und Bestimmen des Zeitpunkts der Detektion,
- Detektieren derselben Pulswelle durch wenigstens einen der zweiten Pulswellensensoren (2) an der jeweiligen zweiten Stelle und Bestimmen des Zeitpunkts der Detektion,
- Erzeugen wenigstens eines akustischen Signals durch wenigstens einen der akustischen Aktoren (3) an der jeweiligen dritten Stelle und Bestimmen des Zeitpunkts der Erzeugung,
- Detektieren desselben akustischen Signals durch wenigstens einen der akustischen Sensoren (4) an der jeweiligen vierten Stelle und Bestimmen des Zeitpunkts der Detektion,
- Bestimmen einer Laufzeit wenigstens einer der Pulswellen zwischen wenigstens einer der ersten und wenigstens einer der zweiten Stellen,
- Bestimmen einer Laufzeit wenigstens eines der akustischen Signale zwischen wenigstens einer der dritten und wenigstens einer der vierten Stellen,
- Bestimmen eines Signallaufwegs des wenigstens einen akustischen Signals zwischen der wenigstens einen dritten Stelle und der wenigstens einen vierten Stelle aus der Laufzeit des wenigstens einen akustischen Signals,
- Bestimmen einer Pulswellengeschwindigkeit aus dem Signallaufweg des wenigstens einen akustischen Signals und der Laufzeit der wenigstens einen Pulswelle.

2. Messverfahren nach Anspruch 1, **dadurch gekennzeichnet, dass** wenigstens zwei erste Pulswellensensoren (1) und/oder wenigstens zwei zweite Pulswellensensoren (2) vorgesehen sind.

3. Messverfahren nach einem der vorhergehenden Ansprüche, **dadurch gekennzeichnet, dass** wenigstens zwei akustische Aktoren (3) und/oder wenigstens zwei akustische Sensoren (4) vorgesehen sind.

4. Messverfahren nach einem der vorhergehenden Ansprüche, **dadurch gekennzeichnet, dass** wenigstens eine der ersten und wenigstens eine der dritten Stellen benachbart sind oder zusammenfallen und/oder dass wenigstens eine der zweiten und wenigstens eine der vierten Stellen benachbart sind oder zusammenfallen.

5. Messverfahren nach einem der vorhergehenden Ansprüche, **dadurch gekennzeichnet, dass** die Detektion der Pulswelle durch wenigstens einen der ersten Pulswellensensoren (1) und/oder durch wenigstens einen der zweiten Pulswellensensoren (2) durch eine Bioimpedanzmessung erfolgt.

6. Messverfahren nach einem der vorhergehenden Ansprüche, **dadurch gekennzeichnet, dass** wenigstens eine der ersten Stellen in der Herzregion, insbesondere im Bereich des Aortenbogens (9), der Messperson (5) liegt.

7. Messverfahren nach einem der vorhergehenden Ansprüche, **dadurch gekennzeichnet, dass** wenigstens eine der zweiten Stellen im Bereich eines Oberschenkels der Messperson (5) liegt.

8. Messvorrichtung zur nicht-invasiven Messung der aortalen Pulswellengeschwindigkeit einer Messperson (5), d. h. der Ausbreitungsgeschwindigkeit von Druckwellen im Blut der Aorta (8) der Messperson (5), mit:
- wenigstens einem ersten Pulswellensensor (1) zur Anbringung an jeweils einer ersten Stelle auf der Hautoberfläche der Messperson (5) im Bereich der Aorta (8), welche von der senkrechten Projektion der Aorta (8) auf die Hautoberfläche höchstens 5 cm, bevorzugt höchstens 3 cm und weiter bevorzugt höchstens 1 cm entfernt ist und besonders bevorzugt auf dieser Projektion liegt,
- wenigstens einem zweiten Pulswellensensor (2) zur Anbringung an jeweils einer zweiten Stelle, welche von jeder der ersten Stellen verschieden ist, auf der Hautoberfläche der Messperson (5) im Bereich der Aorta (8), welche von der senkrechten Projektion der Aorta (8) auf die Hautoberfläche höchstens 5 cm, bevorzugt höchstens 3 cm und weiter bevorzugt höchstens 1 cm entfernt ist und besonders bevorzugt auf dieser Projektion liegt,
- wenigstens einem akustischen Aktor (3) zur Erzeugung akustischer Signale zur Anbringung an jeweils einer dritten Stelle, welche an oder im Bereich wenigstens einer der ersten Stellen liegt, d. h. welche von der betreffenden ersten Stelle höchstens 5 cm, bevorzugt höchstens 3 cm und weiter bevorzugt höchstens 1 cm entfernt ist und besonders bevorzugt mit dieser ersten Stelle zusammenfällt,
- wenigstens einem akustischen Sensor (4) zur Detektion akustischer Signale zur Anbringung an jeweils einer vierten Stelle, welche an oder im Bereich wenigstens einer der zweiten Stellen liegt, d. h. welche von der betreffenden zweiten Stelle höchstens 5 cm, bevorzugt höchstens 3 cm und weiter bevorzugt höchstens 1 cm entfernt ist und besonders bevorzugt mit dieser zweiten Stelle zusammenfällt,
- einer ersten Auswerteeinrichtung (6) zur Bestimmung der Zeitpunkts einer Detektion wenigstens einer Pulswelle durch wenigstens einen der ersten Pulswellensensoren (1) an der jeweiligen ersten Stelle,
- einer zweiten Auswerteeinrichtung (6) zur Bestimmung der Zeitpunkts einer Detektion derselben Pulswelle durch wenigstens einen der zweiten Pulswellensensoren (2) an der jeweiligen zweiten Stelle,
- einer dritten Auswerteeinrichtung (6) zur Bestimmung des Zeitpunkts einer Erzeugung wenigstens eines akustischen Signals durch wenigstens einen der akustischen Aktoren (3) an der jeweiligen dritten Stelle,
- einer vierten Auswerteeinrichtung (6) zur Bestimmung des Zeitpunkts einer Detektion desselben akustischen Signals durch wenigstens einen der akustischen Sensoren (4) an der jeweiligen vierten Stelle,
- einer ersten Laufzeitbestimmungseinrichtung (6) zur Bestimmung einer Laufzeit wenigstens einer der Pulswellen zwischen wenigstens einer der ersten und wenigstens einer der zweiten Stellen,
- einer zweiten Laufzeitbestimmungseinrichtung (6) zur Bestimmung einer Laufzeit wenigstens eines der akustischen Signale zwischen wenigstens einer der dritten und wenigstens einer der vierten Stellen,
- einer Signallaufwegsbestimmungseinrichtung (6) zur Bestimmung eines Signallaufwegs des wenigstens einen akustischen Signals zwischen der wenigstens einen dritten und der wenigstens einen vierten Stelle aus der Laufzeit des wenigstens einen akustischen Signals,
- einer Pulswellengeschwindigkeitsbestimmungseinrichtung (6) zur Bestimmung einer Pulswellengeschwindigkeit aus dem Signallaufweg des wenigstens einen akustischen Signals und der Laufzeit der wenigstens einen Pulswelle.

9. Messvorrichtung nach Anspruch 8, **dadurch gekennzeichnet, dass** wenigstens zwei erste Pulswellensensoren (1) und/oder wenigstens zwei zweite Pulswellensensoren (2) vorgesehen sind.

10. Messvorrichtung nach einem der Ansprüche 8 bis 9, **dadurch gekennzeichnet, dass** wenigstens zwei akustische Aktoren (3) und/oder wenigstens zwei akustische Sensoren (4) vorgesehen sind.

11. Messvorrichtung nach einem der Ansprüche 8 bis 10, **dadurch gekennzeichnet, dass** wenigstens ein erster Pulswellensensor (1) und/oder wenigstens ein zweiter Pulswellensensor (2) als Elektrode, insbesondere als Textil- oder Kunststoffelektrode, ausgebildet ist.

12. Messvorrichtung nach einem der Ansprüche 8 bis 11, **dadurch gekennzeichnet, dass** wenigstens ein erster Pulswellensensor (1) und wenigstens ein akustischer Aktor (3) und/oder dass wenigstens ein zweiter Pulswellensensor (2) und wenigstens ein akustischer Sensor (4) jeweils zur gemeinsamen Anbringung auf der Hautoberfläche der Messperson (5) ausgebildet sind.

13. Messvorrichtung nach einem der Ansprüche 8 bis 12, **dadurch gekennzeichnet, dass** wenigstens einer der ersten Pulswellensensoren (1) und/oder wenigstens einer der zweiten Pulswellensensoren (2) ein Bioimpedanzsensor ist.

## Claims

1. Measuring method for non-invasive measurement of the aortic pulse wave velocity of a measurement subject (5), i.e. the speed of propagation of pressure waves in the blood of the aorta (8) of the measurement subject (5) with the steps of:
- attaching at least one first pulse wave sensor (1) to a respective first location on the skin surface of the measurement subject (5) in the area of the aorta (8), the first location being at most 5 cm away from the orthogonal projection of the aorta (8) onto the skin surface, preferably at most 3 cm away and more preferably at most 1 cm away, and particularly preferably lying on this projection,
- attaching at least one second pulse wave sensor (2) to a respective second location, which differs from each of the first locations, on the skin surface of the measurement subject (5) in the area of the aorta (8), the second location being at most 5 cm away from the orthogonal projection of the aorta (8) onto the skin surface, preferably at most 3 cm away and more preferably at most 1 cm away, and particularly preferably lying on this projection,
- attaching at least one acoustic actuator (3) for generating acoustic signals to a respective third location, which is located at or in the area of at least one of the first locations, the third location being from this first location at most 5 cm away, preferably at most 3 cm away and more preferably at most 1 cm away and particularly preferably coinciding with this first location,
- attaching at least one acoustic sensor (4) for the detection of acoustic signals to a respective fourth location, which is located at or in the area of at least one of the second locations, the fourth location being from this second location at most 5 cm away, preferably at most 3 cm away and more preferably at most 1 cm away and particularly preferably coinciding with this second location,
- detecting at least one pulse wave in the aorta (8) of the measurement subject (5) by at least one of the first pulse wave sensors (1) at the respective first location and determining the point of time of the detection,
- detecting the same pulse wave by at least one of the second pulse wave sensors (2) at the respective second location and determining the point of time of the detection,
- generating at least one acoustic signal by at least one of the acoustic actuators (3) at the respective third location and determining the point of time of the generation,
- detecting the same acoustic signal by at least one of the acoustic sensors (4) at the respective fourth location and determining the point of time of the detection,
- determining a transit time of at least one of the pulse waves between at least one of the first and at least one of the second locations,
- determining a transit time of at least one of the acoustic signals between at least one of the third and at least one of the fourth locations,
- determining a signal path of the at least one acoustic signal between the at least one third location and the at least one fourth location from the transit time of the at least one acoustic signal,
- determining a pulse wave velocity from the signal path of the at least one acoustic signal and the transit time of the at least one pulse wave.

2. Measuring method according to claim 1, **characterized in that** at least two first pulse wave sensors (1) and/or at least two second pulse wave sensors (2) are provided.

3. Measuring method according to one of the preceding claims, **characterized in that** at least two acoustic actuators (3) and/or at least two acoustic sensors (4) are provided.

4. Measuring method according to one of the preceding claims, **characterized in that** at least one of the first and at least one of the third locations are adjacent or coincide and/or that at least one of the second and at least one of the fourth locations are adjacent or coincide.

5. Measuring method according to one of the preceding claims, **characterized in that** the pulse wave is detected by at least one of the first pulse wave sensors (1) and/or by at least one of the second pulse wave sensors (2) by means of a bioimpedance measurement.

6. Measuring method according to one of the preceding claims, **characterized in that** at least one of the first locations lies in the heart region, in particular in the region of the aortic arch (9), of the measurement subject (5).

7. Measuring method according to one of the preceding claims, **characterized in that** at least one of the second locations lies in the region of a thigh of the measurement subject (5).

8. Measuring device for non-invasive measurement of the aortic pulse wave velocity of a measurement subject (5), i.e. the speed of propagation of pressure waves in the blood of the aorta (8) of the measuring subject (5), with:
- at least one first pulse wave sensor (1) for attachment to a respective first location on the skin surface of the measurement subject (5) in the area of the aorta (8), the first location being at most 5 cm away from the orthogonal projection of the aorta (8) onto the skin surface, preferably at most 3 cm away and further preferably at most 1 cm away and particularly preferably lying on this projection,
- at least one second pulse wave sensor (2) for attachment to a respective second location, which differs from each of the first locations, on the skin surface of the measurement subject (5) in the area of the aorta (8), the second location being at most 5 cm away from the orthogonal projection of the aorta (8) onto the skin surface, preferably at most 3 cm away and more preferably at most 1 cm away, and particularly preferably lying on this projection,
- at least one acoustic actuator (3) for generating acoustic signals for attachment to a respective third location, which is located at or in the area of at least one of the first locations, i. e. which is from this first location at most 5 cm away, preferably at most 3 cm away and more preferably at most 1 cm away and particularly preferably coincides with this first location,
- at least one acoustic sensor (4) for the detection of acoustic signals for attachment to a respective fourth location, which is located at or in the area of at least one of the second locations, i. e. which is from this second location at most 5 cm away, preferably at most 3 cm away and more preferably at most 1 cm away and particularly preferably coincides with this second location,
- a first evaluation device (6) for determining the point of time of detection of at least one pulse wave by at least one of the first pulse wave sensors (1) at the respective first location,
- a second evaluation device (6) for determining the point of time of detection of the same pulse wave by at least one of the second pulse wave sensors (2) at the respective second location,
- a third evaluation device (6) for determining the point of time of generation of at least one acoustic signal by at least one of the acoustic actuators (3) at the respective third location,
- a fourth evaluation device (6) for determining the point of time of detection of the same acoustic signal by at least one of the acoustic sensors (4) at the respective fourth location,
- a first transit time determination device (6) for determining a transit time of at least one of the pulse waves between at least one of the first and at least one of the second locations,
- a second transit time determination device (6) for determining a transit time of at least one of the acoustic signals between at least one of the third and at least one of the fourth locations,
- a signal path determination device (6) for determining a signal path of the at least one acoustic signal between the at least one third and the at least one fourth location from the transit time of the at least one acoustic signal,
- a pulse wave velocity determination device (6) for determining a pulse wave velocity from the signal path of the at least one acoustic signal and the transit time of the at least one pulse wave.

9. Measuring device according to claim 8, **characterized in that** at least two first pulse wave sensors (1) and/or at least two second pulse wave sensors (2) are provided.

10. Measuring device according to one of claims 8 to 9, **characterized in that** at least two acoustic actuators (3) and/or at least two acoustic sensors (4) are provided.

11. Measuring device according to one of claims 8 to 10, **characterized in that** at least one first pulse wave sensor (1) and/or at least one second pulse wave sensor (2) is designed as an electrode, in particular as a textile or plastic electrode.

12. Measuring device according to one of claims 8 to 11, **characterized in that** at least one first pulse wave sensor (1) and at least one acoustic actuator (3) and/or that at least one second pulse wave sensor (2) and at least one acoustic sensor (4) are each designed for joint attachment on the skin surface of the measurement subject (5).

13. Measuring device according to one of claims 8 to 12, **characterized in that** at least one of the first pulse wave sensors (1) and/or at least one of the second pulse wave sensors (2) is a bioimpedance sensor.

## Revendications

1. Procédé de mesure servant à la mesure non invasive de la vitesse d'onde pulsée dans l'aorte d'un sujet de mesure (5), en d'autres termes de la vitesse de propagation d'ondes de pression dans le sang de l'aorte (8) du sujet de mesure (5), avec les étapes :
- d'installation d'au moins un premier capteur d'onde pulsée (1) au niveau de respectivement un premier emplacement sur la surface de la peau du sujet de mesure (5) dans la zone de l'aorte (8), dans lequel le premier emplacement est éloigné de la projection perpendiculaire de l'aorte (8) sur la surface de la peau de 5 cm au maximum, de manière préférée de 3 cm au maximum et de manière davantage préférée de 1 cm au maximum et se situe de manière particulièrement préférée sur ladite projection,
- d'installation d'au moins un deuxième capteur d'onde pulsée (2) au niveau de respectivement un deuxième emplacement, lequel est différent de chacun des premiers emplacements, sur la surface de la peau du sujet de mesure (5) dans la zone de l'aorte (8), dans lequel le deuxième emplacement est éloigné de la projection perpendiculaire de l'aorte (8) sur la surface de la peau de 5 cm au maximum, de manière préférée de 3 cm au maximum et de manière davantage préférée de 1 cm au maximum et se situe de manière particulièrement préférée sur ladite projection,
- d'installation d'au moins un actionneur (3) acoustique servant à la génération de signaux acoustiques au niveau de respectivement un troisième emplacement, lequel se situe au niveau de ou dans la zone d'au moins un des premiers emplacements, dans lequel le troisième emplacement est éloigné du premier emplacement concerné de 5 cm au maximum, de manière préférée de 3 cm au maximum et de manière davantage préférée de 1 cm au maximum et de manière particulièrement préférée coïncide avec ledit premier emplacement,
- d'installation d'au moins un capteur (4) acoustique servant à la détection de signaux acoustiques au niveau de respectivement un quatrième emplacement, lequel se situe au niveau de ou dans la zone d'au moins un des deuxièmes emplacements, dans lequel le quatrième emplacement est éloigné du deuxième emplacement concerné de 5 cm au maximum, de manière préférée de 3 cm au maximum et de manière davantage préférée de 1 cm au maximum et de manière particulièrement préférée coïncide avec ledit deuxième emplacement,
- de détection d'au moins une onde pulsée dans l'aorte (8) du sujet de mesure (5) par au moins un des premiers capteurs d'onde pulsée (1) au niveau du premier emplacement respectif et de définition du moment de la détection,
- de détection de la même onde pulsée par au moins un des deuxièmes capteurs d'onde pulsée (2) au niveau du deuxième emplacement respectif et de définition du moment de la détection,
- de génération d'au moins un signal acoustique par au moins un des actionneurs (3) acoustiques au niveau du troisième emplacement respectif et de définition du moment de la génération,
- de détection du même signal acoustique par au moins un des capteurs (4) acoustiques au niveau du quatrième emplacement respectif et de définition du moment de la détection,
- de définition d'un temps de propagation d'une des ondes pulsées entre au moins un des premiers et au moins un des deuxièmes emplacements,
- de définition d'un temps de propagation d'au moins un des signaux acoustiques entre au moins un des troisièmes et au moins un des quatrièmes emplacements,
- de définition d'un trajet de signal de l'au moins un signal acoustique entre l'au moins un troisième emplacement et l'au moins un quatrième emplacement à partir du temps de propagation de l'au moins un signal acoustique,
- de définition d'une vitesse d'onde pulsée à partir du trajet de signal de l'au moins un signal acoustique et du temps de propagation de l'au moins une onde pulsée.

2. Procédé de mesure selon la revendication 1, **caractérisé en ce qu'**au moins deux premiers capteurs d'onde pulsée (1) et/ou au moins deux deuxièmes capteurs d'onde pulsée (2) sont prévus.

3. Procédé de mesure selon l'une quelconque des revendications précédentes, **caractérisé en ce qu'**au moins deux actionneurs (3) acoustiques et/ou au moins deux capteurs (4) acoustiques sont prévus.

4. Procédé de mesure selon l'une quelconque des revendications précédentes, **caractérisé en ce qu'**au moins un des premiers et au moins un des troisièmes emplacements sont adjacents ou coïncident, et/ou qu'au moins un des deuxièmes et au moins un des quatrièmes emplacements sont adjacents ou coïncident.

5. Procédé de mesure selon l'une quelconque des revendications précédentes, **caractérisé en ce que** la détection de l'onde pulsée est effectuée par au moins un des premiers capteurs d'onde pulsée (1) et/ou par au moins un des deuxièmes capteurs d'onde pulsée (2) par une mesure de bio-impédance.

6. Procédé de mesure selon l'une quelconque des revendications précédentes, **caractérisé en ce qu'**au moins un des premiers emplacements se situe dans la région du cœur, en particulier dans la région de la crosse aortique, en particulier dans la zone de la crosse aortique (9), du sujet de mesure (5).

7. Procédé de mesure selon l'une quelconque des revendications précédentes, **caractérisé en ce qu'**au moins un des deuxièmes emplacements se situe dans la zone d'une cuisse du sujet de mesure (5).

8. Dispositif de mesure servant à la mesure non invasive de la vitesse d'onde pulsée de l'aorte d'un sujet de mesure (5), en d'autres termes de la vitesse de propagation d'ondes de pression dans le sang de l'aorte (8) du sujet de mesure (5), avec
- au moins un premier capteur d'onde pulsée (1) destiné à être installé au niveau de respectivement un premier emplacement sur la surface de la peau du sujet de mesure (5) dans la zone de l'aorte (8), lequel est éloigné de la projection perpendiculaire de l'aorte (8) sur la surface de la peau de 5 cm au maximum, de manière préférée de 3 cm au maximum et de manière davantage préférée de 1 cm au maximum et se situe de manière particulièrement préférée sur ladite projection,
- au moins un deuxième capteur d'onde pulsée (2) destiné à être installé au niveau de respectivement un deuxième emplacement, lequel est différent de chacun des premiers emplacements, sur la surface de la peau du sujet de mesure (5) dans la zone de l'aorte (8), lequel est éloigné de la projection perpendiculaire de l'aorte (8) sur la surface de la peau de 5 cm au maximum, de manière préférée de 3 cm au maximum et de manière davantage préférée de 1 cm au maximum et se situe de manière particulièrement préférée sur ladite projection,
- au moins un actionneur (3) acoustique servant à la génération de signaux acoustiques, destiné à être installé au niveau de respectivement un troisième emplacement, lequel se situe au niveau de ou dans la zone d'au moins un des premiers emplacements, en d'autres termes lequel est éloigné du premier emplacement concerné de 5 cm au maximum, de manière préférée de 3 cm au maximum et de manière davantage préférée de 1 cm au maximum et de manière particulièrement préférée coïncide avec ledit premier emplacement,
- au moins un capteur (4) acoustique servant à la détection de signaux acoustiques, destiné à être installé au niveau de respectivement un quatrième emplacement, lequel se situe au niveau de ou dans la zone d'au moins un des deuxièmes emplacements, en d'autres termes lequel est éloigné du deuxième emplacement concerné de 5 cm au maximum, de manière préférée de 3 cm au maximum et de manière davantage préférée de 1 cm au maximum et de manière particulièrement préférée coïncide avec ledit deuxième emplacement,
- un premier système d'évaluation (6) servant à la définition du moment d'une détection d'au moins une onde pulsée par au moins un des premiers capteurs d'onde pulsée (1) au niveau du premier emplacement respectif,
- un deuxième système d'évaluation (6) servant à la définition du moment d'une détection de la même onde pulsée par au moins un des deuxièmes capteurs d'onde pulsée (2) au niveau du deuxième emplacement respectif,
- un troisième système d'évaluation (6) servant à la définition du moment d'une génération d'au moins un signal acoustique par au moins un des actionneurs (3) acoustiques au niveau du troisième emplacement respectif,
- un quatrième système d'évaluation (6) servant à la définition du moment d'une détection du même signal acoustique par au moins un des capteurs (4) acoustiques au niveau du quatrième emplacement respectif,
- un premier système de définition de temps de propagation (6) servant à la définition d'un temps de propagation d'au moins une des ondes pulsées entre au moins un des premiers et au moins un des deuxièmes emplacements,
- un deuxième système de définition de temps de propagation (6) servant à la définition d'un temps de propagation d'au moins un des signaux acoustiques entre au moins un des troisièmes et au moins un des quatrièmes emplacements,
- un système de définition de trajet de signal (6) servant à la définition d'un trajet de signal de l'au moins un signal acoustique entre l'au moins un troisième et l'au moins un quatrième emplacement à partir du temps de propagation de l'au moins un signal acoustique,
- un système de définition de vitesse d'onde pulsée (6) servant à la définition d'une vitesse d'onde pulsée à partir du trajet de signal de l'au moins un signal acoustique et du temps de propagation de l'au moins une onde pulsée.

9. Dispositif de mesure selon la revendication 8, **caractérisé en ce qu'**au moins deux premiers capteurs d'onde pulsée (1) et/ou au moins deux capteurs d'onde pulsée (2) sont prévus.

10. Dispositif de mesure selon l'une quelconque des revendications 8 à 9, **caractérisé en ce qu'**au moins deux actionneurs (3) acoustiques et/ou au moins deux capteurs (4) acoustiques sont prévus.

11. Dispositif de mesure selon l'une quelconque des revendications 8 à 10, **caractérisé en ce qu'**au moins un premier capteur d'onde pulsée (1) et/ou au moins un deuxième capteur d'onde pulsée (2) sont réalisés en tant qu'électrode, en particulier en tant qu'électrode en textile ou en matière plastique.

12. Dispositif de mesure selon l'une quelconque des revendications 8 à 11, **caractérisé en ce qu'**au moins un premier capteur d'onde pulsée (1) et au moins un actionneur (3) acoustique et/ou au moins un deuxième capteur d'onde pulsée (2) et au moins un capteur (4) acoustique sont réalisés respectivement pour être installés conjointement sur la surface de la peau du sujet de mesure (5).

13. Dispositif de mesure selon l'une quelconque des revendications 8 à 12, **caractérisé en ce qu'**au moins un des premiers capteurs d'onde pulsée (1) et/ou au moins un des deuxièmes capteurs d'onde pulsée (2) sont un capteur de bio-impédance.
